(19) Europäisches Patentamt
European Patent Office
Office européen des brevets

(11) Veröffentlichungsnummer: **0 000 194**
**B1**

(12) # EUROPÄISCHE PATENTSCHRIFT

(21) Anmeldenummer: **78100239.9**

(22) Anmeldetag: **26.06.78**

(51) Int. Cl.³: **C 07 C 127/22**

(54) **Verfahren zur Herstellung von Isocyanatgruppen aufweisenden Allophanaten und ihre Verwendung zur Herstellung von Lackierungen**

(30) Priorität: **02.07.77 DE 2729990**

(43) Veröffentlichungstag der Anmeldung:
**10.01.79 Patentblatt 79/01**

(45) Bekanntmachung des Hinweises auf die Patenterteilung:
**23.07.80 Patentblatt 80/15**

(84) Benannte Vertragsstaaten:
**BE DE FR GB NL SE**

(56) Entgegenhaltungen:
**The Journal of Organic Chemistry, Band 26, Seite 3004 - 3005 (1961)**

(73) Patentinhaber: **Bayer AG
Zentralbereich Patente, Marken und Lizenzen
D - 5090 Leverkusen 1, Bayerwerk (DE)**

(72) Erfinder: **König, Klaus, Dr.
Heymannstrasse 50
D - 5090 Leverkusen (DE)
Reichmann, Wolfgang, Dr.
Vohwinkelallee 19
D - 4000 Düsseldorf (DE)
Pedain, Josef, Dr.
Haferkamp 6
D - 5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**EP 0 000 194 B1**

# Verfahren zur Herstellung von Isocyanatgruppen aufweisenden Allophanaten und ihre Verwendung zur Herstellung von Lackierungen

In der britischen Patentschrift Nr. 994 890 wird ein Verfahren zur Herstellung von organischen Polyisocyanaten beschrieben, bei dem Urethanisocyanate der allgemeinen Formel

$$R'(O—CO—NH—R—NCC)_n$$

in der

R' einen mono- oder polyvalenten organischen Rest einer mono- oder polyfunktionellen Hydroxylverbindung,

n eine ganze Zahl von 1 bis 6 und

R einen bifunktionellen organischen Rest bedeutet,

mit überschüssigen Diisocyanaten entweder rein thermisch oder in Gegenwart von Metallcarboxylaten, Metallchelaten oder tertiären Aminen als Katalysator zur Reaktion gebracht werden, bis der eintretende NCO-Abfall einer vollständigen Umsetzung der vorhandenen Urethangruppen mit Isocyanatgruppen entspricht. Die exakte Konstitution der Reaktionsprodukte kann gemäß der Lehre der britischen Patentschrift nicht mit Sicherheit angegeben werden. Aus den gemessenen NCO-Werten der Reaktionsgemische bzw. der daraus isolierten Endprodukte wird geschlossen, daß diese im wesentlichen Allophanatpolyisocyanate darstellen. Bei der genaueren analytischen Kontrolle der nach der Lehre der britischen Patentschrift erhaltenen Produkte durch IR-Spektroskopie und insbesondere gelchromatographische Untersuchung wird jedoch gefunden, daß ein beträchtlicher Teil aus Isocyanuratpolyisocyanaten und Uretdionpolyisocyanaten besteht, die durch als Nebenreaktionen ablaufende Dimerisierung und Trimerisierung der NCO-Gruppen entstehen. Wird nun die Reaktion nach Erreichen des für vollständige Allophanatisierung errechneten NCO-Gehaltes abgestoppt, so bleibt für jede durch Nebenreaktion abreagierte NCO-Gruppe eine Urethangruppe unumgesetzt im Reaktionsgemisch zurück.

Das Auftreten von Trimerisierung und Dimerisierung als Nebenreaktionen bei den Umsetzungen nach der Lehre der britischen Patentschrift 994 890 ist nicht überraschend, da bei Abwesenheit von Katalysatoren bis zum Abfall des NCO-Gehaltes auf den für vollständigen Umsatz der Urethangruppen berechneten Wert lange Reaktionszeiten bei relativ hohen Temperaturen (z.B. 24 Stunden bei 130 bis 135°C in Beispiel 1, Seite 3, Zeilen 49 bis 51) benötigt werden und die Bildung von Isocyanuraten aus Allophanaten und Isocyanaten bzw. Dimerer dieser Isocyanate literaturbekannt ist (J. C. Kogon, Journ. Am. Chem. Soc., Vol. 78, 1956, Seiten 4911 bis 4914).

Die Verwendung von Katalysatoren gestattet zwar eine starke Erniedrigung der Reaktionstemperatur (s. 2, Zeilen 92 bis 95), jedoch ist seit langem bekannt, daß die beschriebenen Katalysatoren (Metallcarboxylate, Metallchelate, tertiäre Amine) hervorragende Dimerisierungs- und Trimerisierungskatalysatoren für Isocyanate sind, so daß das Auftreten solcher Nebenreaktionen in beträchtlichem Ausmaß bei der Umsetzung von Urethangruppen mit Isocyanaten zu Allophanaten durchaus erklärlich wird.

Die gemäß Verfahren der GB—PS 994 890 nicht auszuschließenden Nebenreaktionen zu dimeren bzw. trimeren Polyisocyanaten führen zu Reaktionsgemischen, die sich von den entsprechenden reinen Allophanat-Polyisocyanaten insbesondere durch eine schlechtere Verträglichkeit mit zahlreichen Polyhydroxylverbindungen, insbesondere mit Polyhydroxypolyacrylaten, wie sie bei der Herstellung von Polyurethankunststoffen als Reaktionspartner für die Polyisocyanate herangezogen werden, unterscheiden.

Das Problem der Herstellung von reinen Allophanat-Polyisocyanaten, d.h. von Allophanat-Polyisocyanaten, welche nicht mit dimeren und insbesondere trimeren Polyisocyanaten "verunreinigt" sind wurde auch schon in den DT—AS'en 2 009 179 bzw. 2 040 645 angesprochen. Bei den Verfahren dieser Veröffentlichungen geht es jedoch um die Herstellung von aromatische gebundene Isocyanatgruppen aufweisenden Allophanatpolyisocyanaten, die gemäß der Lehre der genannten Vorveröffentlichungen dann frei von den genannten Nebenprodukten erhalten werden können, wenn die zum Allophanatpolyisocyanat führende Additionsreaktion in Gegenwart von alkylierend wirkenden Substanzen (DT—AS 2 009 179), sowie gegebenenfalls in Gegenwart von bestimmten Metallverbindungen als Katalysatoren (DT—AS 2 040 645) durchgeführt wird. Die Verfahren der DT—AS'en 2 009 179 und 2 040 645 sind jedoch für die Herstellung von Allophanatpolyisocyanaten mit aliphatisch und/oder cycloaliphatisch gebundenen Isocyanatgruppen ungeeignet. So sinkt der Isocyanatgehalt eines Reaktionsgemischs aus einem Urethan und einem aliphatischen Polyisocyanat bei 160—170°C während 50 Stunden nur unwesentlich ab. Im Falle der gleichzeitigen Mitverwendung von Metallkatalysatoren gemäß DT—AS 2 040 645 wurde bei 110—120°C im Verlauf von 35 Stunden nur eine 66 %ige Umsetzung beobachtet. Das Reaktionsgemisch hatte sich dabei sehr stark verfärbt. Gemäß Stand der Technik steht somit kein brauchbares Verfahren zur Herstellung von reinen, hellfarbigen Allophanatpolyisocyanaten mit aliphatisch oder cycloaliphatisch gebundenen Isocyanatgruppen zur Verfügung.

Es war Aufgabe der vorliegenden Erfindung,

ein derartiges Verfahren zur Verfügung zu stellen.

Überraschenderweise konnte diese Aufgabe dadurch gelöst werden, daß man die Umsetzung zwischen aliphatischen bzw. cycloaliphatischen Polyisocyanaten und Urethangruppen aufweisenden Verbindungen in Gegenwart von bestimmten, nachstehend näher beschriebenen Säuren durchführt.

Gegenstand der vorliegenden Erfindung ist ein Verfahren zur Herstellung von aliphatisch und/oder cycloaliphatisch gebundene Isocyanatgruppen aufweisenden Allophanaten durch Umsetzung von Urethangruppen aufweisenden organischen Verbindungen mit organischen Polyisocyanaten mit aliphatisch und/oder cycloaliphatisch gebundenen Isocyanatgruppen, dadurch gekennzeichnet, daß man die Umsetzung in Gegenwart von starken, mit aliphatischen bzw. cycloaliphatischen Isocyanaten ein gemischtes Carbamidsäureanhydrid bildenden Säuren durchführt.

Gegenstand der vorliegenden Erfindung ist auch die Verwendung der nach diesem Verfahren erhaltenen, Isocyanatgruppen aufweisenden Allophanate in Kombination mit hydroxylfunktionellen höhermolekularen Verbindungen zur Herstellung von Lackierungen.

Beim erfindungsgemäßen Verfahren werdenals Ausgangsmaterialien beliebige Urethangruppen und gegebenenfalls aliphatisch oder cycloaliphatisch gebundene Isocyanatgruppen aufweisende, ansonsten unter den Reaktionsbedingungen inerte organische Verbindungen eingesetzt. Diese Verbindungen werden im allgemeinen durch Umsetzung von Isocyanaten mit alkoholischen oder auch phenolischen Hydroxylgruppen aufweisenden Verbindungen erhalten. Es ist jedoch auch möglich, beim erfindungsgemäßen Verfahren solche Urethane einzusetzen, die beispielsweise durch Umsetzung von Chlorameisensäureestern mit primäre Aminogruppen aufweisenden Aminen oder auf beliebig anderem Wege erhalten worden sind. Gemäß einer besonderen Ausführungsform des erfindungsgemäßen Verfahrens werden als Ausgangsmaterialien in situ aus Phenolen oder Alkoholen und überschüssigen Mengen an aliphatischen bzw. cycloaliphatischen Polyisocyanaten hergestellte Urethane eingesetzt. Das bei dieser Umsetzung erhaltene Reaktionsgemisch enthält dann bereits die zweite Hauptkomponente des erfindungsgemäßen Verfahrens, das aliphatische bzw. cycloaliphatische Polyisocyanat, welches bei der Urethanherstellung im Überschuß eingesetzt worden war. Zu den bevorzugten, beim erfindungsgemäßen Verfahren als Ausgangsmaterialien einzusetzenden Urethangruppen aufweisenden Verbindungen gehören solche der allgemeinen Formel

$$A{-}(O{-}\overset{\overset{O}{\|}}{C}{-}NH{-}R_1{-}NCO)_n$$

in welcher

A für einen Rest steht, wie er durch Entfernung der Hydroxylgruppen aus einer n-wertigen organischen Hydroxylgruppen aufweisenden Verbindung erhalten wird, welcher von den Hydroxylgruppen abgesehen gegenüber Isocyanatgruppen inert ist,

$R_1$ für einen Rest steht, wie er durch Entfernung der Isocyanatgruppen aus einem Diisocyanat mit aliphatisch und/oder cycloaliphatisch gebundenen Isocyanatgruppen erhalten wird und

n für eine ganze Zahl von 1 bis 4 steht.

Entsprechend diesen Ausführungen werden die bevorzugt beim erfindungsgemäßen Verfahren einzusetzenden, Isocyanatgruppen aufweisenden Urethane der genannten allgemeinen Formel vorzugsweise durch Umsetzung von Hydroxylgruppen aufweisenden Verbindungen der Formel

$$A(OH)_n$$

mit Diisocyanaten der Formel

$$R_1(NCO)_2$$

erhalten, wobei pro Mol der Hydroxylgruppen aufweisenden Verbindung vorzugsweise mindestens n Mol des Diisocyanats zum Einsatz gelangen. Es ist jedoch auch möglich, bei dieser, zu den Urethangruppen aufweisenden Ausgangsmaterialien des erfindungsgemäßen Verfahrens führenden Umsetzung pro Mol der Hydroxylgruppen aufweisenden Verbindung $A(OH)_n$ weniger als n Mole eines Diisocyanats einzusetzen, d.h. die Menge des Diisocyanats so zu bemessen, daß sie im Bereich zwischen 0,5 n und 1 n Mol pro Mol der Hydroxylgruppen aufweisenden Verbindung liegt. In diesem Falle würden wegen der eintretenden Kettenverlängerungsreaktion über Urethangruppen mehr als n Urethangruppen aufweisende Ausgangsmaterialien entstehen. Ebenfalls möglich wäre der Einsatz von Urethangruppen aufweisenden Verbindungen, die durch Umsetzung von Hydroxylgruppen aufweisenden Verbindungen $A(OH)_n$ mit Monoisocyanaten und/oder höher als difunktionellen Polyisocyanaten gegebenenfalls im Gemisch mit Diisocyanaten erhalten worden sind, und welche gegebenenfalls keine freien Isocyanatgruppen aufweisen.

Die Herstellung der Urethangruppen aufweisenden Ausgangsmaterialien für das erfindungsgemäße Verfahren erfolgt nach den altbekannten Methoden der Polyurethanchemie, d.h. insbesondere durch einfaches Erhitzen der Ausgangsmaterialien auf 40 bis 150°, vorzugsweise 50 bis 100°C.

Als Polyhydroxylverbindungen $A(OH)_n$ können sowohl Phenole wie z.B. Phenol, $\alpha$-Naphthol, Kresol, Resorcin oder Tris-hydroxybenzole als auch alkoholische Hydroxylgruppen aufweisende organische Verbindungen eingesetzt werden. Derartige alkoholische Hydroxylgruppen aufweisende Verbindungen

sind gegenüber den beispielhaft genannten Phenolen bevorzugt.

Zu diesen bevorzugten alkoholischen Hydroxylverbindungen $A(OH)_n$ gehören

1. Niedermolekulare, gegebenenfalls Ätherbrücken aufweisende 1-4-wertige aliphatische Alkohole des Molekulargewichtsbereichs 32—250 wie z.B. Methanol, Äthanol, Propanol, Isopropanol, isomere Butanole, Allylalkohol, Pentanole, Hexanole und Heptanole, 2-Äthylhexanol, Fettalkohole mit 10—20 Kohlenstoffatomen, Äthandiol, Propandiol-1,2 und -1,3, Butandiol-1,2, -1,3 und -1,4, Pentandiol-1,5, Neopentylglykol, Hexandiol-1,6 und -2,5, 3-Methylpentandiol-1,5, 2-Methyl-2-propylpropandiol-1,3, 2,2-Diäthyl-propandiol-1,3, 2-Äthylhexandiol-1,3, 2,2,4-Trimethylpentandiol-1,3, Trimethylhexandiol-1,6, Decandiol-1,10, Dodecandiol-1,12, 2-Butandiol-1,4, 2-Methylenpropandiol-1,3, Glyzerin, Butantriol, 2-Hydroxymethyl-2-methylpropandiol-1,3, 1,2,6-Hexantriol, Trimethylol-äthan, Trimethylolpropan, Pentaerythrit, Äthylenglykol-monoalkyl- oder -aryläther, Propylenglykol-monoalkyläther, Diäthylenglykol, Triäthylenglykol, Tetraäthylenglykol.

2. Cycloaliphatische 1-4-wertige Alkohole des Molekulargewichtsbereichs 88—250, wie z.B. Cyclopentanol, Cyclohexanol, Methylcyclohexanol, Trimethylcyclohexanol, 4-tert.-Butylcyclohexanol, Menthol, Borneol und Isoborneol, 2-Hydroxydecalin, 1,2-, 1,3- und 1,4-Cyclohexandiol, 2,4-Dihydroxy-1,1,3,3-tetramethylcyclobutan, 1,4-Bis-hydroxymethylcyclohexan, Bis-(4-hydroxycyclohexyl)-methan, 2,2-Bis(4-hydroxycyclohexyl)-propan, 2-Methyl-2,4-bis-(4-hydroxycyclohexyl)-pentan, Furfuryl- und Tetrahydrofurfurylalkohol, Bis-hydroxymethylnorbornan, Dihydroxymethyl-tricyclodecan.

3. Araliphatische 1-4-wertige Alkohole des Molekulargewichtsbereichs 103—300, wie z.B. Benzylalkohol, Phenyläthylalkohol, 3-Phenylpropanol oder 4,4'-Di-(2-hydroxyäthyl)-diphenylmethan oder.

4. 1-4 Hydroxylgruppen aufweisende Polythioäther, Polyacetale, Polycarbonate oder insbesondere Polyester bzw. Polyäther der in der Polyurethanchemie an sich bekannten Art mit mittleren Molekulargewichten von 250 bis 5000, vorzugsweise 300 bis 2000. Die in Frage kommenden Hydroxylgruppen aufweisenden Polyester sind z.B. Umsetzungsprodukte von mehrwertigen, vorzugsweise zweiwertigen und gegebenenfalls zusätzlich dreiwertigen Alkoholen mit mehrwertigen, vorzugsweise zweiwertigen, Carbonsäuren. Anstelle der freien Polycarbonsäuren können auch die entsprechenden Polycarbonsäureanhydride oder entsprechende Polycarbonsäureester von niedrigen Alkoholen oder deren Gemische zur Herstellung der Polyester verwendet werden. Die Polycarbonsäuren können aliphatischer, cycloaliphatischer, aromatischer und/oder heterocyclischer Natur sein und gegebenenfalls, z.B. durch Halogenatome, substituiert und/oder ungesättigt sein. Als Beispiele hierfür seien genannt:

Bernsteinsäure, Adipinsäure, Korksäure, Azelainsäure, Sebacinsäure, Phthalsäure, Isophthalsäure, Trimellitsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Fumarsäure, dimere und trimere Fettsäuren wie Ölsäure, gegebenenfalls in Mischung mit monomeren Fettsäuren, Terephthalsäuredimethylester, Terephthalsäure-bis-glykolester. Als mehrwertige Alkohole kommen z.B. Äthylenglykol, Propylenglykol-(1,2) und -(1,3), Butylenglykol-(1,4) und -(2,3), Hexandiol-(1,6), Octandiol-(1,8), Neopentylglykol, Cyclohexandimethanol(1,4 - bis - hydroxymethylcyclohexan), 2-Methyl-1,3-propandiol, Glycerin, Trimethylolpropan, Hexantriol-(1,2,6), Butantriol-(1,2,4), Trimethyloläthan, Pentaerythrit, Chinit, Mannit und Sorbit, Methylglykosid, ferner Diäthylenglykol, Triäthylenglykol, Tetraäthylenglykol, Polyäthylenglykole, Dipropylenglykol, Polypropylenglykole, Dibutylenglykol und Polybutylenglykole in Frage. Auch Polyester aus Lactonen, z.B. $\varepsilon$-Caprolacton oder Hydroxycarbonsäuren, z.B. $\omega$-Hydroxycapronsäure, sind einsetzbar.

Auch die erfindungsgemäß in Frage kommenden eine bis vier Hydroxylgruppen aufweisenden Polyäther sind solche der an sich bekannten At und werden z.B. durch Polymerisation von Epoxiden wie Äthylenoxid, Propylenoxid, Butylenoxid, Tetrahydrofuran, Styroloxid oder Epichlorhydrin mit sich selbst, z.B. in Gegenwart von $BF_3$, oder durch Anlagerung dieser Epoxide, gegebenenfalls im Gemisch oder nacheinander, an Startkomponenten mit reaktionsfähigen Wasserstoffatomen wie Alkohole oder Phenole, z.B. Wasser, Äthylenglykol, Propylenglykol-(1,3) oder -(1,2), Trimethylolpropan, 4,4'-Dihydroxydiphenylpropan hergestellt.

Unter den Polythioäthern seien insbesondere die Kondensationsprodukte von Thiodiglykol mit sich selbst und/oder mit anderen Glykolen, Dicarbonsäuren oder Formaldehyd angeführt. Je nach den Co-Komponenten handelt es sich bei den Produkten um Polythiomischäther, Polythioätherester oder Polythioäther-polyacetale.

Als Polyacetale kommen z.B. die aus Glykolen, wie Diäthylenglykol, Triäthylenglykol, 4,4' - Dioxäthoxy - diphenyldimethylmethan, Hexandiol und Formaldehyd herstellbaren Verbindungen in Frage. Auch durch polymerisation cyclischer Acetale lassen sich erfindungsgemäß geeignete Polyacetale herstellen.

Als Hydroxylgruppen aufweisende Polycarbonate kommen solche der an sich bekannten Art in Betracht, die z.B. durch Umsetzung von Diolen wie Propandiol-(1,3), Butandiol-(1,4) und/oder Hexandiol-(1,6), Diäthylenglykol, Triäthylenglykol, Tetraäthylenglykol mit Diarylcarbonaten, z.B. Diphenylcarbonat oder Phosgen, hergestellt werden können.

Die unter 1. genannten einfachen aliphatischen Alkohole, sowie die unter 4. genannten Polyester- bzw. Polyätherpolyole werden beim erfindungsgemäßen Verfahren bevorzugt eingesetzt.

Selbstverständlich können auch Mischungen der vorstehend genannten Hydroxylverbindungen eingesetzt werden. Dies ist sogar eine bevorzugte Ausführungsform des erfindungsgemäßen Verfahrens, da durch den Einsatz eines Gemisches von Hydroxylverbindungen unterschiedlicher Funktionalität, die Funktionalität des Allophanatgruppen enthaltenden Polyisocyanats in gewünschter Weise variiert werden kann.

Zur Herstellung der als Ausgangsmaterialien des erfindungsgemäßen Verfahrens dienenden Urethangruppen aufweisenden Verbindungen, sowie als Reaktionspartner für diese Urethangruppen aufweisenden Verbindungen kommen erfindungsgemäß vorzugsweise Diisocyanate der Formel

$$R_2(NCO)_2$$

zum Einsatz, wobei

$R_2$ für einen aliphatischen Kohlenwasserstoffrest mit 2 bis 20, vorzugsweise 6 bis 10, Kohlenstoffatomen, einen cycloaliphatischen Kohlenwasserstoffrest mit 4 bis 20, vorzugsweise 6 bis 15 Kohlenstoffatomen oder einen Xylylenrest steht.

Beispiele derartiger Isocyanate sind Äthylendiisocyanat, Tetramethylendiisocyanat, Hexamethylendiisocyanat, Undecamethylendiisocyanat, 2,4,4 - Trimethyl - 1,6 - diisocyanatohexan, 3 - Isocyanatomethyl - 3,5,5 - trimethyl - cyclohexylisocyanat, 1,3 - Diisocyanato-cyclobutan, 1,4 - Diisocyanato-cyclohexan, 4,4' - Diisocyanato - dicyclohexylmethan, 1,2 - Bis - (isocyanatomethyl) - cyclobutan, Trimethylhexan - 1,6 - diisocyanat, 1,11 - Diisocyanatoundecan, 3 - Isocyanatomethyl - 3,5,5 - trimethyl - cyclohexylisocyanat, 4,4' - Cyclohexandiisocyanat, 4,4' - Dicyclohexylmethan-diisocyanat, 1,2 - Bis - (isocyanatomethyl)-cyclobutan, Bis - isocyanatomethylnorbornan (Isomerengemisch), 3(4), 8(9) - Diisocyanatomethyltricyclo - $(5.2.1.0^{2.6})$-decan oder p-Xylylendiisocyanat. Sowohl bei der Herstellung der erfindungsgemäß einzusetzenden Urethangruppen aufweisenden Verbindungen als auch als deren Reaktionspartner werden derartige Diisocyanate eingesetzt. Besonders bevorzugt ist Hexamethylendiisocyanat.

Bei der Herstellung der als Ausgangsmaterialien dienenden Urethangruppen aufweisenden Verbindungen, nicht jedoch als deren Reaktionspartner beim erfindungsgemäß Verfahren können auch Monoisocyanate wie z.B. n-Hexylisocyanat oder Cyclohexylisocyanat verwendet bzw. mitverwendet werden, obwohl dies weniger bevorzugt ist.

Sowohl bei der Herstellung der als Ausgangsmaterialien dienenden Urethangruppen aufweisenden Verbindungen als auch als deren Reaktionspartner können auch höher als difunktionelle aliphatische bzw. cycloaliphatische Polyisocyanate verwendet bzw. mitverwendet werden. Beispiele derartiger Polyisocyanate sind die Isocyanuratgruppen aufweisenden Trimerisierungsprodukte von Hexamethylendiisocyanat oder 3-Isocyanatomethyl-3,5,5-trimethyl-cyclohexylisocyanat.

Sowohl bei der Herstellung der Urethangruppen aufweisenden Ausgangsmaterialien als auch als deren Reaktionspartner können beliebige Gemische der genannten Isocyanate eingesetzt werden, mit der Einschränkung, daß man als Reaktionspartner für die Urethangruppen aufweisenden Verbindungen zweckmäßigerweise auf die Mitverwendung von Monoisocyanaten verzichten wird, da bei deren Mitverwendung die NCO-Funktionalität der erfindungsgemäßen Verfahrensprodukte herabgesetzt würde. Durch Wahl bestimmter Mischungsverhältnisse der Isocyanatkomponente kann ebenso wie durch Wahl des Mischungsverhältnisses verschiedener Hydroxylverbindungen die Funktionalität der erfindungsgemäßen Verfahrensprodukte variiert werden.

Erfindungswesentlich ist die Mitverwendung von Säuren bei der Umsetzung der Urethangruppen aufweisenden Verbindungen mit der Isocyanat-Komponente zu den entsprechenden Isocyanatgruppen aufweisenden Allophanaten. Bei diesen Säuren handelt es sich um Protonen abspaltende starke Säuren, welche mit aliphatischen oder cycloaliphatischen Isocyanaten unter Bildung eines gemischten Säureanhydrids reagieren, wobei die dem Isocyanat entsprechende Carbamidsäure und die Protonen abspaltende Säure die Säuren des gemischten Säureanhydrids darstellen. So reagieren derartige, für das erfindungsgemäße Verfahren geeignete Säuren HX (X = Säurerest nach Abspaltung des Protons) mit Isocyanaten Y—NCO zu Addukten der Formel Y—NH—CO—X, welche als gemischtes Anhydrid der Carbamidsäure Y—NH—COOH und der Säure HX anzusehen sind. Beispiele geeigneter Säuren sind. Halogenwasserstoffe, wie z.B. Fluorwasserstoff, Chlorwasserstoff, Bromwasserstoff oder Jodwasserstoff, Chlorsulfonsäure, Fluorsulfonsäure, Schwefelsäure, Alkansulfonsäuren wie z.B. Methansulfonsäure oder perhalogenierte Alkansulfonsäuren wie z.B. Trifluormethansulfonsäure. Chlorwasserstoff ist die beim erfindungsgemäßen Verfahren bevorzugt einzusetzende Säure.

Die Säuren werden beim erfindungsgemäßen Verfahren in Mengen von 0,001—10,0, vorzugsweise 0,01—1,0 Gew.-% bezogen auf das Gesamtgewicht der Reaktionspartner eingesetzt.

Die Säuren können dem Reaktionsgemisch

nach beliebigen Methoden einverleibt werden. So ist es beispielsweise möglich, die Säure bereits der Hydroxylgruppen aufweisenden Verbindung vor Herstellung der Urethangruppen aufweisenden Verbindung zuzumischen, eine Variante, die eine bequeme Ausführungsform des Verfahrens insbesondere bei Verwendung von Chlorwasserstoff darstellt, da Chlorwasserstoff in vielen Hydroxylgruppen aufweisenden Verbindungen leicht löslich ist und man somit auf das Einleiten kleiner Mengen gasförmigen Chlorwasserstoffs verzichten kann. Es ist jedoch auch möglich, die Säure zusammen mit der Isocyanat-Komponente entweder bereits bei der Bildung der Urethangruppen aufweisenden Verbindungen oder, falls im Zweistufenverfahren gearbeitet wird, bei der Herstellung der Allophanatgruppen aufweisenden Verbindungen aus separat hergestellter Urethangruppen aufweisender Verbindung und einer Polyisocyanat-Komponente zuzumischen. Selbstverständlich kann als Katalysator auch das Carbamidsäurechlorid, das sich von dem eingesetzen oder von einem anderen Isocyanat ableitet, eingesetzt werden. Auf eine zusätzliche Katalyse kann gegebenenfalls verzichtet werden, wenn als Diisocyanat Rohdestillate verwendet werden, die noch einen Restgehalt an hydrolisierbarem Chlor von über 0,001 Gew.-%, vorwiegend in Form des Carbamidsäurechlorids, enthalten.

Zur Durchführung des erfindungsgemäßen Verfahrens werden im allgemeinen die Reaktionspartner in solchen Mengen eingesetzt, daß auf jede Urethangruppe der Urethangruppen aufweisenden Verbindung 2 bis 50, vorzugsweise 3 bis 12 Isocyanatgruppen der Polyisocyanat-Komponente, vorzugsweise des Diisocyanats entfallen. Im Falle der Herstellung der Urethangruppen aufweisenden Verbindung in situ wird dementsprechend ein entsprechender Überschuß der Isocyanat-Komponente, vorzugsweise Diisocyanat-Komponente, eingesetzt.

Die erfindungsgemäße Umsetzung erfolgt im allgemeinen im Temperaturbereich zwischen 90 und 140°C. Der Verlauf der erfindungsgemäßen Umsetzung kann durch Bestimmung des NCO-Gehalts des Reaktionsgemisches verfolgt werden. Die Reaktion kann zu jedem beliebigen Zeitpunkt beispielsweise durch Abkühlen auf Raumtemperatur oder durch Entfernung der katalytisch wirkenden Säure beispielsweise durch Anlegen von Vakuum abgebrochen werden. Diese letztgenannte Methode ist insbesondere bei Verwendung von gasförmigen Säuren möglich. Auch eine Desaktivierung des Säure-Katalysators durch Zugabe von Verbindungen, welche mit den Säuren unter Adduktbildung reagieren (beispielsweise Propylenoxid oder eine aktive ungesättigte Verbindung wie z.B. Styrol ist möglich.

Bei der bevorzugten Variante des erfindungsgemäßen Verfahrens unter Herstellung der Urethangruppen aufweisenden Ausgangsverbindung in situ wird im allgemeinen wie folgt verfahren:

Das vorzugsweise als Isocyanat-Komponente eingesetzte Diisocyanat wird bei 50—80°C vorgelegt und die Hydroxyl-Komponente in flüssiger Form unter gutem Rühren eingetropft. Soll für die Urethanbildung und die Allophanatbildung das gleiche Isocyanat bzw. Isocyanatgemisch verwendet werden, so setzt man es am einfachsten von Anfang an in einem solchen Überschuß ein, daß das NCO/OH-Verhältnis etwa zwischen 3:1 und 12:1 liegt.

Nach erfolgter Urethanreaktion — kontrolliert durch Bestimmung des NCO-Gehaltes — wird der Katalysator (im allgemeinen Chlorwasserstoff) zugegeben. Nun wird die Temperatur auf 90—140°C gesteigert und so lange gerührt, bis der NCO-Gehalt auf den für vollständige Allophanatisierung errechneten Wert abgesunken ist.

Bei Temperaturen von über 130°C ist es zweckmäßig, die Apparatur unter gelindem Überdruck zu halten, damit der Chlorwasserstoff nicht entweicht. Der Katalysator kann jedoch auch zusammen mit dem Isocyanat vorgelegt werden, oder gemeinsam mit der Hydroxylverbindung eindosiert werden.

Nach beendeter Reaktion kann der Katalysator in einfacher Weise durch Andestillieren im Vakuum entfernt oder durch Zugabe äquivalenter Mengen Propylenoxid oder einer aktiven ungesättigten Verbindung addiert werden. Falls das Allophanatgruppen enthaltende Polyisocyanat von überschüssigem Diisocyanat befreit werden soll, geschieht dies entweder durch Dünnschichtdestillation oder durch fraktionierte Extraktion, beispielsweise unter Verwendung von n-Hexan oder Cyclohexan als Extraktionsmittel. Im ersten Fall kann der Chlorwasserstoff im Rohprodukt belassen werden, weil er zusammen mit dem Diisocyanat abdestilliert und hinterher im Destillat enthalten ist. Dieses kann anschließend für einen erneuten Ansatz verwendet werden.

Bei der Durchführung des erfindungsgemäßen Verfahrens werden im allgemeinen Art und Mengenverhältnisse der Ausgangsmaterialien so gewählt, daß mindestens zwei Isocyanatgruppen aufweisende Allophanate, d.h. Allophanatpolyisocyanate als Verfahrensprodukte entstehen. Derartige erfindungsgemäße Verfahrensprodukte zeichnen sich durch eine hervorragende Stabilität während der Dünnschichtbehandlung auch bei Temperaturen von 180°C und mehr aus. Es treten nicht die bei Polyisocyanaten mit Biuretstruktur beobachteten Neben- und Äquilibrierungsreaktionen ein, die zu lästigen Anbackungen und zur Erhöhung der Viskosität führen.

Das etfindungsgemäße Verfahren eignet sich für eine kontinuierlich Durchführung. Hierbei werden zweckmäßigerweise mehrere Reaktoren in Form einer Kaskade hintereinandergeschaltet. In den ersten Reaktor werden kontinuierlich Diisocyanat, Hydroxylverbindung und Katalysator eindosiert. Durch Einstellung von Temperatur und Durchsatz wird erreicht, daß die Reaktion bei Verlassen des letzten Reaktors vollständig ist. Das Rohprodukt durchläuft anschließend einen Dünnschichtverdampfer, wo es von überschüssigem Diisocyanat befreit wird. Dieses wird in den ersten Reaktor zurückgeführt.

Die erfindungsgemäß herstellbaren Allophanatpolyisocyanate können gegebenenfalls nach Entfernung überschüssigen Diisocyanats zur Herstellung Polyurethan-Schaumstoffen, Elastomeren, Duromeren, Beschichtungen und Verklebungen eingesetzt werden.

Besonders geeignet sind sie als Rohstoffe für hochwertige, lichtstabile, wetterfeste Lackierungen eventuell in Kombination mit hydroxylfunktionellen höhermolekularen Verbindungen. Hierbei zeichnen sich die Allophanatpolyisocyanate im Vergleich zu Polyisocyanaten mit Urethan-, Biuret- oder Isocyanuratstruktur durch ihre gute Verträglichkeit mit handelsüblichen Polyacrylaten aus.

Ein weiterer Vorteil des erfindungsgemäßen Verfahrens ist in den vielfältigen Variationsmöglichkeiten insbesondere bezüglich Art und Mengenverhältnis der preiswerten Ausgangsmaterialien (Hydroxylgruppen aufweisende Verbindungen) zu sehen. So kann z.B. die NCO-Funktionalität der erfindungsgemäßen Verfahrensprodukte je nach Wahl der Hydroxylverbindung in weiten Grenzen gesteuert werden. Der Einsatz von Fettalkoholen ergibt Produkte mit guter Benzinlöslichkeit. Durch Verwendung von cycloaliphatischen oder aromatischen Hydroxylverbindungen wird eine hervorragende Härte der Lackierungen erreicht.

Insbesondere ist auch die ausgezeichnete Lagerstabilität der erfindungsgemäßen, von überschüssigem Ausgangsisocyanat befreiten, Allophanatpolyisocyanate hervorzuheben. Die erfindungsgemäßen Verfahrensprodukte zeigen keinerlei Tendenze zur Abspaltung von monomerem Ausgangsisocyanat und unterscheiden sich insbesondere in diesem Punkt vorteilhaft von den bekannten Biurethgruppen aufweisenden Polyisocyanaten.

Durch folgende Beispiele wird die Erfindung näher erläutert:

Beispiel 1

In einem 3 l-Dreihalskolben wurden zu 2016 g (12 Mol) Hexamethylendiisocyanat bei 70°C im Verlauf von 30 Minuten 150 g (1 Mol) Triäthylenglykol zugetropft. Nach weiteren 30 Minuten bei 70°C betrug der NCO-Gehalt der Reaktionsmischung 42,65%, was vollständiger Umsetzung der OH-Gruppen zu Urethangruppen entspricht. Nun wurden 7 g Chlorwasserstoff eingeleitet und die Temperatur auf 100°C erhöht. Nach 8,5 Stunden entsprach der NCO-Gehalt der Reaktionsmischung mit 38,8% einer vollständigen Umsetzung der Urethangruppen zu Allophanatgruppen. Das Rohprodukt wurde einer Dünnschichtdestillation unterworfen. Es wurden 900 g eines fahlgelben Produktes mit einer Viskosität von 1200 cP/25°C und einen NCO-Gehalt von 19,3% erhalten. Das Präparat wurde gelchromatographisch auf seine Zusammensetzung untersucht (Tabelle 1).

*Vergleichsbeispiele analog GB—PS 994 890*

A) (ohne Katalysator wie in Beispiel 1 des britischen Patents)

Wie im vorstehenden Beispiel 1 wurde zunächst eine Lösung des Bisurethans in überschüssigem Hexamethylendiisocyanat hergestellt. Ein weiterer Abfall des NCO-Gehaltes bis zu dem für vollständige Allophanatisierung berechneten Wert von 38,8% konnte erst nach 48 Stunden bei 135-145°C erreicht werden. Das Gemisch hatte sich in dieser Zeit stark verfärbt. Das Produkt wurde wie in Beispiel 1 aufgearbeitet und analysiert (Tabelle 1).

Viskosität: 1330 cP/25°C;
NCO-Gehalt: 19,5%.

B) (Zink-Naphthenat als Katalysator wie in Beispiel 5 der GB—PS)

Wie in vorstehendem Beispiel 1 wurde die Urethanlösung bereitet und dann mit 2,3 g Zinknaphthenat versetzt. Im Verlauf von 8 Stunden bei 50°C sank der NCO-Gehalt auf 38,8%. Nach Aufarbeitung: (Analyse Tabelle 1)

Viskosität: 1350 cP/25°C;
NCO-Gehalt: 20,4%;
Farbe: braungelb.

C) (Tertiäres Amin als Katalysator wie in Beispiel 4 der GB—PS)

Nach Zusatz von 2,3 g Diazabicyclooctan zur Urethanlösung wurde zunächst 24 Stunden auf 70°C geheizt. Der theoretische NCO-Abfall wurde aber erst nach weiteren 16 Stunden bei 120°C erreicht. Nach Aufarbeitung wurde ein tiefgelbes Öl der Viskosität von 1050 cP/25°C mit einem NCO-Gehalt von 20,2% erhalten (Analyse Tabelle 1).

TABELLE 1

(Gelchromatographische Analyse)

| Komponente (Gew.%) | Beispiel 1 | Vergleichsbeispiele A | Vergleichsbeispiele B | Vergleichsbeispiele C |
|---|---|---|---|---|
| Hexamethylendiisocyanat | 0,5 | 0,6 | 0,7 | 0,5 |
| Dimeres Diisocyanat | 1,5 | 5,8 | 1,5 | 6,3 |
| Trimeres Diisocyanat | 1,0 | 7,3 | 10,6 | 11,3 |
| Bisurethan aus 1 Mol Triäthylenglykol und 2 Mol Diisocyanat | – | 6,8 | 10,3 | 10,3 |
| Monourethan-Monoallophanat aus 1 Mol Triäthylenglykol und 3 Mol Diisocyanat | 7,0 | 18,3 | 9,8 | 15,6 |
| Bisallophanat aus 1 Mol Triäthylenglykol und 4 Mol Diisocyanat | 44,7 | 16,5 | 20,8 | 12,2 |
| Summe aller Polymer-homologer Verbindungen | 45,3 | 44,7 | 46,3 | 43,8 |

Vergleichsbeispiele analog DAS 2 009 178 und 2 040 645, in denen die Herstellung aromatischer Allophanat-Polyisocyanate beschrieben wird.

D) Aus 1008 g (6 Mol) Hexamethylendiisocyanat und 106 g (1 Mol) Diäthylenglykol wurde zunächst bei 70°C eine Urethanlösung bereitet (NCO-Gehalt: 36,85%). Nach Zugabe von 0,7 g p-Toluolsulfonsäuremethylester wurde unter Überleiten von trockenem Stickstoff die Temperatur auf 160°C gesteigert. Nach 50 Stunden war der NCO-Gehalt auf 33,5% gefallen (berechnet für vollständige Allophanatisierung 30,1%). Da das Reaktionsgemisch sehr stark verfärbt war, wurde der Ansatz zu diesem Zeitpunkt abgebrochen. Eine IR-spektroskopische Analyse ergab einen deutlichen Gehalt an Uretdion- und Isocyanuratgruppen.

E) Aus 1008 g (6 Mol) Hexamethylendiisocyanat und 57 g (0,75 Mol) 1,2-Propylenglykol wurde bei 70°C eine Urethanlösung bereitet (NCO-Gehalt: 41,4%). Nach Zugabe von 1 g p-Toluolsulfonsäuremethylester und 0,2 g Zinkacetylacetonat wurde die Temperatur allmählich auf 110—120°C gesteigert. Im Verlauf von 36 Stunden fiel der NCO-Gehalt der Reaktionsmischung auf 38,1% ab (ber.: für vollständige Allophanatisierung 35,45%). Der Ansatz war gelbbraun verfärbt; ein IR-Spektrum zeigte eine starke Isocyanurat-Bande.

Bei dem Versuch, die NCO-Abnahme bei 150—160°C zu vervollständigen, trat plötzlich unter exothermer Reaktion und Gasentwicklung eine vollständige Verquallung des Reaktionsgemisches ein.

Beispiel 2

1512 g (9 Mol) Hexamethylendiisocyanat wurden bei 100°C mit 120 g (0,5 Mol) aufgeschmolzenem 2,2-Bis(4-hydroxycyclohexyl)-propan versetzt. Anschließend wurde ebenfalls bei 100°C eine Lösung von 4 g Chlorwasserstoff in 16 g (0,5 Mol) Methanol zugetropft. Nach weiteren 9 Stunden unter Stickstoff bei 100°C betrug der NCO-Gehalt 38,27% (berechnet für vollständige Allophanatbildung 38,4%). Der Ansatz wurde anschließend mit 8 g Propylenoxid bei 50°C 15 Minuten lang verrührt. Danach war kein hydrolysierbares Chlor mehr festzustellen. Nach Entgasen im Vakuum wurde das Rohprodukt der Dünnschichtdestillation bei 170°C/0,5 mm unterworfen. Man erhielt 630 g eines farblosen Öls mit einer Viskosität von 11.600 cP/25°C und einem NCO-Gehalt von 17,4%. Der Restgehalt an Hexamethylendiisocyanat betrug 0,48%.

Beispiel 3

In 3024 g (18 Mol) Hexamethylendiisocyanat wurden bei 90°C 4 g Chlorwasserstoff eingeleitet. Anschließend tropfte man 180 g (2

Mol) 1,4-Butandiol zu. Nach einer weiteren Stunde wurde die Temperatur auf 100°C erhöht. Nach 8 Stunden unter Stickstoff war der NCO-Gehalt der Mischung auf den für vollständige Allophanatbildung errechneten Wert von 36,8% gefallen. Nach Verrühren mit 10 g Propylenoxid bei 50°C wurde das Produkt im Vakuum entgast und der Destillation im Dünnschichtverdampfer unterworfen. Man erhielt 1415 g einer blaßgelben Flüssigkeit. (Destillat: 1750 g Hexamethylendiisocyanat).
Viskosität: 6500 cP/25°C;
NCO-Gehalt: 18,6%;
Restgehalt an Diisocyanat: 0,65%.

Das IR-Spektrum zeigte Spuren von Uretdion- und keine Isocyanuratgruppen an. Eine Probe des Produkts wurde 50 Tage lang bei 50°C gelagert. Danach wurde der Restgehalt an Hexamethylendiisocyanat zu 0,66% ermittelt.

Beispiel 4

Analog Beispiel 1 wurden aus 3024 g (18 Mol) Hexamethylendiisocyanat, 201 g (1,5 Mol) Trimethylolpropan und 8 g Bromwasserstoff als Katalysator 1480 g eines hochviskosen, hochfunktionellen Allophanatpolyisocyanats hergestellt, das 80%ig in Xylol/Äthylglykolacetat (1:1) gelöst eine Viskosität von 2800 cP/25°C und einen NCO-Gehalt von 18,2% hatte.

Beispiel 5

2150 g (1 Mol) eines auf n-Butanol gestarteten Polyäthers aus 80 Mol-% Äthylenoxid und 20 Mol-% Propylenoxid wurden mit 1008 g (6 Mol) Hexamethylendiisocyanat und 4 g Chlorwasserstoff versetzt. Nach 12 Stunden bei 110°C war die Allophanatreaktion abgeschlossen. Man erhielt nach Dünnschichtbehandlung 2300 g eines blaßgelben Öls der Viskosität 1200 cP/25°C mit einem NCO-Gehalt von '3,2%, das nach einiger Zeit wachsartig erstarrte.

Beispiel 6

Analog Beispiel 1 wurden aus 3024 g (18 Mol) Hexamethylendiisocyanat, 261 g (4,5 Mol) Allylalkohol und 7 g Chlorwasserstoff als Katalysator ein bifunktionelles Allophanat mit einer Viskosität von 200 cP/25°C und einem NCO-Gehalt von 17,8% hergestellt.

Beispiel 7

228 g (1 Mol) 2,2-Bis-(4-hydroxyphenyl)-propan wurden aufgeschmolzen und aus einem beheizten Tropftrichter zu 2016 g (12 Mol) Hexamethylendiisocyanat bei 100°C getropft. Anschließend wurden 5 g Chlorwasserstoff eingeleitet und 5 Stunden bei 110°C unter Stickstoff gerührt. Danach war die Allophanatreaktion beendet (NCO-Gehalt: 37,5%). Nach zweimaliger Dünnschichtdestillation erhielt man 730 g eines viskosen Öls (Viskosität: 124 000 cP/25°C), das sich nach einigem Stehen verfestigte (Erweichungspunkt: 45—47°C). Der NCO-Gehalt betrug 16,03%. Der Gehalt an freiem Hexamethylendiisocyanat von 0,8% stieg bei der 30-tagigen Lagerung bei 50°C nicht an (0,78%).

Beispiel 8

1332 g (6 Mol) 3-Isocyanatomethyl-3,5,5-trimethyl-cyclohexylisocyanat-1 wurden bei 100°C mit einem Gemisch aus 59 g (0,5 Mol) Hexandiol-1,6 und 37 g n-Butanol versetzt. Nach einer Stunde wurden 18 g Chlorwasserstoff eingeleitet und die Temperatur auf 120°C gesteigert. Nach 18 Stunden war die Allophanatreaktion beendet (NCO-Gehalt 27,6%). Nach Dünnschichtdestillation bei 190°C und 0,4 mm erhielt man ein helles Harz, das 80%ig in Äthylglykolacetate gelöst eine Viskosität von 890 cP bei 25°C und einen NCO-Gehalt von 10,2% hatte.

Beispiel 9

Durch Reaktion von 630 g (3 Mol) 2,4,4-Trimethyl-1,6-diisocyanat mit 30 g (0,3 Mol) Butandiol-1,4 und 1,5 g Chlorwasserstoff während 6 Stunden bei 110°C und anschließende Dünnschichtdestillation wurden 360 g eines hochviskosen farblosen Öls erhalten.
Viskosität: 150 000 cP/25°C;
NCO-Gehalt: 15,3%.

Beispiel 10

Aus 678 g (3 Mol) 6-Isocyanatohexansäure-2-isocyanatoäthylester, 39 g Hexandiol-1,6 (0,3 Mol) und 1,5 g Chlorwasserstoff wurde in 5 Stunden bei 110°C ein Rohprodukt hergestellt, das nach Dünnschichtdestillation ein helles Öl der Viskosität 28 000 cP/25°C ergab (NCO-Gehalt: 14,7%).

Beispiel 11

Aus 3024 g (18 Mol) Hexamethylendiisocyanat und 152 g (2 Mol) 1,2-Propandiol wurde bei 70°C eine Urethanlösung in überschüssigem Diisocyanat bereitet. Je 380 g des Gemisches wurden mit verschiedenen Katalysatoren erhitzt, bis der für vollständige Allophanatisierung errechnete NCO-Gehalt erreicht war. Die Ergebnisse sind in der folgenden Tabelle aufgeführt:

TABELLE 2

| Katalysator | Menge | Art der Zugabe | Reaktionszeit | Temperatur |
|---|---|---|---|---|
| Schwefelsäure | 1,0 g | in 5 g Propylenglykol | 5 h | 110°C |
| Chlorsulfonsäure | 1,0 g | in 10 ml Methylenchlorid gelöst | 4 h | 110°C |
| Methansulfonsäure | 0,8 g | in Substanz | 10 h | 110°C |

Die erhaltenen Produkte zeigten im IR-Spektrum keine Isocyanurat- oder Uretdion-Rande.

Beispiel 12
(Verwendungsbeispiel)

154 g einer 65%igen Lösung eines Polyesters aus 6 Mol Phthalsäureanhydrid und 7 Mol Trimethylolpropan (Hydroxylgehalt 8%) in Äthylglycolacetat/Xylol (1:1) wurden nach Zugabe von 1 g eines tertiären Amins als Katalysator und 0,4 g Cellulose-butyrat-propionat als Verlaufmittel mit 230 g eines Lösungsmittelgemisches aus Methyläthylketon, Butylacetat, Äthylglycolacetat und Toluol (4:1:4:1) verdünnt. Hierzu wurden 152 g einer 75%igen Lösung des Polyisocyanats aus Beispiel 2 in Äthylglycolacetat/Xylol (1:1) gegeben (NCO/OH-Molverhältnis = 1:1). Die fertige Lacklösung wurde dann auf Stahlbleche aufgetragen, wo die Lackfilme bei Raumtemperatur aushärteten. Die durchgehärteten Klarlackfilme waren kratzfest, elastisch und gegen Lösungsmittel wie Toluol, Äthylglycolacetat, Äthylacetat oder Aceton beständig. Sie hatten ferner folgende Eigenschaften:

Schichtdicke ca. 50 μ
Erichsentiefung (DIN 53 156)
nach 6 Tagen 8,7 mm
nach 9 Tagen 8,6 mm
Pendelhärte (DIN 53 157)
nach 6 Tagen 238 Sekunden
nach 8 Tagen 220 Sekunden
nach 14 Tagen 227 Sekunden

Beispiel 13
(Verwendungsbeispiel)

Es wurden 154 g der im Beispiel 12 beschriebenen Polyesterlösung mit 100 g Titandioxid (Rutiltyp) zu einer Paste verarbeitet. Dieser Paste wurden neben Katalysator und Verlaufmittel 140 g des schon beschriebenen Lösungsmittelgemisches zugefügt. Die so erhaltene Mischung wurde mit 152 g einer 75%igen Lösung des Polyisocyanats aus Beispiel 12 in Äthylglycolacetat/Xylol (1:1) versetzt und in dünner Schicht auf Stahlbleche aufgetragen. Die pigmenthaltigen Lackfilme härteten bei Raumtemperatur durch. Sie zeichneten sich durch Kratzfestigkeit und Lösungsmittelresistenz aus und hatten verglichen mit den Klarlackfilmen folgende Eigenschaften:

Schichtdicke ca. 50 μ
Erichsentiefung (DIN 53 156)
nach 6 Tagen 8,1 mm
nach 9 Tagen 8,0 mm
Pendelhärte (DIN 53 157)
nach 6 Tagen 193 Sekunden
nach 9 Tagen 187 Sekunden
nach 14 Tagen 192 Sekunden

**Patentansprüche**

1. Verfahren zur Herstellung von aliphatisch und/oder cycloaliphatisch gebundene Isocyanatgruppen aufweisenden Allophanaten durch Umsetzung von Urethangruppen aufweisenden organischen Verbindungen mit organischen Polyisocyanaten mit aliphatisch und/oder cycloaliphatisch gebundenen Isocyanatgruppen, dadurch gekennzeichnet, daß man die Umsetzung in Gegenwart von starken, mit aliphatischen bzw. cycloaliphatischen Isocyanaten ein gemischtes Carbamidsäureanhydrid bildenden Säuren durchführt.

2. Verfahren gemäß Anspruch 1, dadurch gekennzeichnet, daß man die Säuren in einer Menge von 0,001—10 Gew.-%, bezogen auf das Polyisocyanat, einsetzt.

3. Verfahren gemäß Anspruch 1 und 2, dadurch gekennzeichnet, daß man als Säure Halogenwasserstoff verwendet.

4. Verwendung der gemäß Anspruch 1 erhaltenen, Isocyanatgruppen aufweisenden Allophanate in Kombination mit hydroxylfunktionellen höhermolekularen Verbindungen zur Herstellung von Lackierungen.

**Revendications**

1. Procédé de préparation d'allophanates comportant des groupes isocyanate à liaison aliphatique et/ou cycloaliphatique par réaction de composés organiques comportant des groupes uréthane avec des polyisocyanates organiques comportant des groupes isocyanate à liaison aliphatique et/ou cycloaliphatique, caractérisé en ce qu'on effectue la réaction en présence d'acides forts formant un anhydride d'acide carbamique mixte avec des isocyanates aliphatiques ou cycloaliphatiques.

2. Procédé suivant la revendication 1, caractérisé en ce qu'on utilise les acides en une quantité de 0,001 à 10% en poids, rapporté au polyisocyanate.

3. Procédé suivant l'une quelconque des revendications 1 et 2, caractérisé en ce que, comme acide, on utilise un hydracide halogéné.

4. Utilisation des allophanates comportant des groupes isocyanate et obtenus conformément à la revendication 1, en combinaison avec des composés à fonction hydroxy et à poids moléculaire supérieur pour la fabrication de vernis.

**Claims**

1. Process for the preparation of allophanates containing aliphatically and/or cycloaliphatically bound isocyanate groups by the reaction of organic compounds which contain urethane groups with organic polyisocyanates containing aliphatically and/or cycloaliphatically bound isocyanate groups, characterised in that the reaction is carried out in the presence of strong acids which form a mixed carbamic acid anhydride with aliphatic or cycloaliphatic isocyanates.

2. Process according to claim 1, characterised in that the acids are used in a quantity of from 0.001 to 10% by weight, based on the polyisocyanate.

3. Process according to claims 1 and 2, characterised in that the acid used is a hydrogen halide.

4. Use of the allophanates containing isocyanate groups, obtained according to claim 1, in combination with hydroxyl functional higher molecular weight compounds, for the production of lacquers.